# EUROPEAN PATENT APPLICATION

(11) **EP 1 449 846 A1**
(43) Date of publication of application: **25.08.2004**
(21) Application number: 03026772.8
(22) Date of filing: 28.04.2000
(51) Int. Cl.: C07J 43/00, A61K 31/58, A61P 23/00

(54) **3-alpha Hydroxy-3-beta methoxymethyl-21-heterocycle substituted steriods with anaesthetic activity**

(30) Priority: 29.04.1999 US 131578 P
(62) Divisional of application: 00930250.6
(71) Applicant: Euro-Celtique S.A., 2330 Luxembourg (LU)
(72) Inventor: Hogenkamp, Derk j., Carlsbad CA 92008 (US)
(74) Representative: King, Elizabeth Caroline

(57) **Abstract**

This invention relates to compounds having Formula (I) or a pharmaceutically acceptable salt, prodrug or solvate thereof, wherein; R₁ is H or methyl; R₂ is 5α- or 5β- H; R₃ is an optionally substituted *N*-attached heteroaryl group or a group -X-R₄; R₄ is an optionally substituted carbon-attached heteroaryl group; and X is O, S or N. The invention also is directed to the use of 3α- hydroxy-3β-methoxymethyl-substituted steroids as sedative/hypnotics and for inducing anesthesia.

## Description

### Background of the Invention

### Field of the Invention

The present invention relates to the field of medicinal chemistry and to novel steroid derivatives and methods for modulating brain excitability. More specifically, the invention relates to 3α-hydroxy-3β-methoxymethyl-21-substituted-5α- (and 5β-)pregnan-20-ones with properties desirable for use as sedative/hypnotics and anesthetics.

### Related Background Art

The naturally occurring neuroactive steroids are unsuitable as sedative/hypnotics because they have poor oral bioavailability presumably due to rapid first-pass metabolism (Hogenkamp. D. J. *et al. J Med. Chem.* 40:61-72 (1997)). The addition of 3β-substitution results in neuroactive steroids that do show potent oral activity in animals but generally last too long to be useful sedative/hypnotics. A sedative/hypnotic should have an elimination half-life in humans < 5 hours to avoid residual next-day effects and accumulation on continued nightly dosing (Nicholson, A. N. *Drugs* 31: 164-176 (1986)). We have found. however. that 3β-methoxymethyl-substituted steroids. while maintaining the oral activity of other 3β-substituted neuroactive steroids, have a duration action that makes them useful as sedative/hypnotics and anesthetics.

Bolger *et al.* in US patent 5,232,917 disclose compounds of the following Formula: 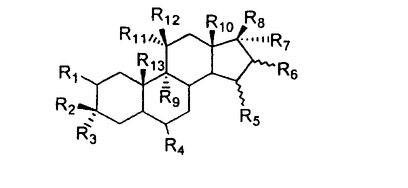 wherein R₁-R₁₃ are individually selected from a large number of groups. The compounds are described as useful as anticonvulsants, sedative/hypnotics and anesthetics.

International Published Application WO 95/21617 discloses compounds of the following Formula: 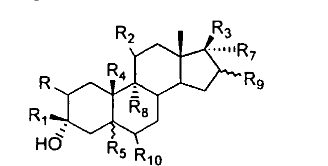 wherein R, R₁-R₁₀ are individually selected from a large number of groups. The compounds are described as useful as anticonvulsants, sedative/hypnotics and anesthetics.

### Summary of the Invention

The present invention is related to 3α-hydroxy-3β-methoxymethyl-21-substituted-5α- (and 5β-pregnan-20-ones with properties especially desirable for use as sedative/hypnotics and anesthetics.

The present invention is also directed to the use of a compound of Formula I as an anesthetic.

A first aspect of the present invention is directed to the novel methoxymethyl-substituted steroids of Formula **I.**

A second aspect of the present invention is directed to the novel compounds of Formula **I** as sedative-hypnotics.

A third aspect of the present invention is to provide a method of inducing anesthesia by administering a compound of Formula **I** to a mammal in need of such treatment.

A fourth aspect of the present invention is to provide a pharmaceutical composition containing an effective amount of a compound of Formula **I** in a mixture with one or more pharmaceutically acceptable carriers or diluents.

### Detailed Description of the Invention

The present invention arises out of the discovery that novel 3β-methoxymethyl-3α-hydroxy-substituted steroids of Formula **I** have duration of action that makes them especially useful as sedative/hypnotics and anesthetics.

The compounds useful in this aspect of the present invention are 3β-methoxymethyl-3α-hydroxy-substituted steroids represented by Formula **I:** 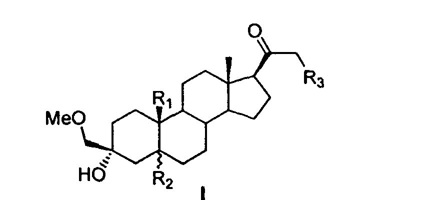 or a pharmaceutically acceptable salt, prodrug or solvate thereof, wherein:
R₁ is H or methyl;
R₂ is 5α- or 5β-H;
R₃ is an optionally substituted *N*-attached heteroaryl group or a group -X-R₄;
R₄ is an optionally substituted-carbon attached heteroaryl group; and
X is O, S or N.

A preferred group of compounds of Formula **I** are compounds where R₄ is an optionally substituted carbon attached bicyclic heteroaryl group; and
X=O.

An additional group of preferred compounds of Formula I are wherein:
R₄ is an optionally substituted carbon attached heteroaryl group; and
X=S.

Another preferred group includes compounds of Formula **I** where R₃ is an optionally substituted *N*-attached monocyclic heteroaryl group. Preferred neuroactive steroids include 3α-hydroxy-3β-methoxymethyl-21-(quinolin-6-yloxy)-5α-pregnan-20-one and 21-(5'-amino-[1,3,4]-thiadiazol-2-ylthio)-3α-hydroxy-3β-methoxymethyl-5α-pregnan-20-one.

A more preferred group of compounds of Formula **I** are compounds where R₄ is the *N*-oxide of an optionally substituted carbon attached bicyclic heteroaryl group; and
X=O.

Other more preferred groups include compounds of Formula I where R₃ is an *N*-attached imidazole or tetrazole that may be optionally substituted.

Especially preferred are the following compounds: 3α-hydroxy-21-(1'-imidazolyl)-3β-methoxymethyl-5α-pregnan-20-one and its hydrochloride salt, 3α-hydroxy-21-(1'-imidazolyl)-3β-methoxymethyl-5β-pregnan-20-one and its hydrochloride salt, 3α-hydroxy-3β-methoxymethyl-21-(2'-tetrazolyl)-5α-pregnan-20-one and 3α-hydroxy-3β-methoxymethyl-21-(quinolin-6-yloxy)-5α-pregnan-20-one, *N*-oxide.

Useful compounds in this aspect of the present invention include without limitation:
3α-hydroxy-21-(1'-imidazolyl)-3β-methoxymethy)-5α-pregnan-20-one;
3α-hydroxy-21-(1'-imidazolyl)-3β-methoxymethyl-5β-pregnan-20-one;
3α-hydroxy-3β-methoxymethyl-21-(2'-tetrazolyl)-5α-pregnan-20-one;
3α-hydroxy-3β-methoxymethyl-21-(quinolin-6-yloxy)-5α-pregnan-20-one, *N*-oxide and
21-(5'-amino-[1,3,4]-thiadiazol-2-ylthio)-3α-hydroxy-3β-methoxymethyl-5α-pregnan-20-one.

Useful aryl groups are C₆₋₁₄ aryl, especially C₆₋₁₀ aryl. Typical C₆₋₁₄ aryl groups include phenyl, naphthyl, phenanthryl, anthracyl, indenyl, azulenyl, biphenyl, biphenylenyl and fluorenyl groups.

Useful cycloalkyl groups are C₃₋₈ cycloalkyl. Typical cycloalkyl groups include cyclopropyl, cyclobutyl. cyclopentyl and cyclohexyl and cycloheptyl.

Useful saturated or partially saturated carbocyclic groups are cycloalkyl groups as defined above, as well as cycloalkenyl groups, such as cyclopentenyl, cycloheptenyl and cyclooctenyl.

Useful heteroaryl groups include any one of the following: thienyl, benzo[b]thienyl, naphtho[2,3-b]thienyl, thianthrenyl, furyl, pyranyl, isobenzofuranyl, chromenyl, xanthenyl, phenoxanthiinyl, 2*H*-pyrrolyl, pyrrolyl, imidazolyl, pyrazolyl, pyridyl, tetrazolyl, pyrazinyl, pyrimidinyl, pyridazinyl, indolizinyl, isoindolyl, 3*H*-indolyl, indolyl, indazolyl, purinyl, 4*H*-quinolizinyl, isoquinolyl, quinolyl, phthalzinyl, naphthyridinyl, quinozalinyl, cinnolinyl, pteridinyl, carbazolyl, β-carbolinyl, phenanthridinyl, acrindinyl, perimidinyl, phenanthrolinyl, phenazinyl, isothiazolyl, phenothiazinyl, isoxazolyl, furazanyl, phenoxazinyl, thiadiazolyl, 1,4-dihydroquinoxaline-2,3-dione, 7-aminoisocoumarin, pyrido[1,2-a]pyrimidin-4-one, 1,2-benzoisoxazol-3-yl, benzimidazolyl, 2-oxindolyl and 2-oxobenzimidazolyl.

Useful halo or halogen groups include fluorine, chlorine, bromine and iodine.

Useful alkyl groups include straight-chained and branched C₁₋₁₀ alkyl groups, more preferably C₁₋₆ alkyl groups. Typical C₁₋₁₀ alkyl groups include methyl, ethyl, propyl, isopropyl, butyl, *sec*-butyl, *tert*-butyl, 3-pentyl, hexyl and octyl groups. Also contemplated is a trimethylene group substituted on two adjoining positions on the benzene ring of the compounds of the invention.

Useful alkenyl groups are C₂₋₆ alkenyl groups, preferably C₂₋₄ alkenyl. Typical C₂₋₄ alkenyl groups include ethenyl, propenyl, isopropenyl, butenyl, and *sec.*-butenyl.

Useful alkynyl groups are C₂₋₆ alkynyl groups, preferably C₂₋₄ alkynyl. Typical C₂₋₄ alkynyl groups include ethynyl, propynyl. butynyl, and 2-butynyl groups.

Useful arylalkyl groups include any of the above-mentioned C₁₋₁₀ alkyl groups substituted by any of the above-mentioned C₆₋₁₄ aryl groups. Useful values include benzyl, phenethyl and naphthylmethyl.

Useful arylalkenyl groups include any of the above-mentioned C₂₋₄ alkenyl groups substituted by any of the above-mentioned C₆₋₁₄ aryl groups.

Useful arylalkynyl groups include any of the above-mentioned C₂₋₄ alkynyl groups substituted by any of the above-mentioned C₆₋₁₄ aryl groups. Useful values include phenylethynyl and phenylpropynyl.

Useful cycloalkylalkyl groups include any of the above-mentioned C₁₋ ₁₀ alkyl groups substituted by any of the above-mentioned cycloalkyl groups.

Useful haloalkyl groups include C₁₋₁₀ alkyl groups substituted by one or more fluorine, chlorine, bromine or iodine atoms, e.g. fluoromethyl, difluoromethyl, trifluoromethyl, pentafluoroethyl, 1,1-difluoroethyl and trichloromethyl groups.

Useful hydroxyalkyl groups include C₁₋₁₀ alkyl groups substituted by hydroxy, e.g. hydroxymethyl, hydroxyethyl, hydroxypropyl and hydroxybutyl groups.

Useful alkoxy groups include oxygen substituted by one of the C₁₋₁₀ alkyl groups mentioned above.

Useful alkylthio groups include sulfur substituted by one of the C₁₋₁₀ alkyl groups mentioned above.

Useful acylamino groups are any C₁₋₆ acyl (alkanoyl) attached to an amino nitrogen, e.g. acetamido. propionamido, butanoylamido, pentanoylamido, hexanoylamido as well as aryl-substituted C₁₋₆ substituted acyl groups.

Useful acyloxy groups are any C₁₋₆ acyl (alkanoyl) attached to an oxy (-O-) group, e.g. acetoxy, propionoyloxy, butanoyloxy, pentanoyloxy, hexanoyloxy and the like.

Useful saturated or partially saturated heterocyclic groups include tetrahydrofuranyl, pyranyl, piperidinyl, piperizinyl, pyrrolidinyl, imidazolidinyl, imidazolinyl, indolinyl, isoindolinyl, quinuclidinyl, morpholinyl, isochromanyl, chromanyl, pyrazolidinyl pyrazolinyl, tetronoyl and tetramoyl groups.

Useful heterocycloalkyl groups include any of the above-mentioned C₁₋₁₀ alkyl groups substituted by any of the above-mentioned heterocyclic groups.

Useful amino groups include -NH₂, -NHR₅, and -NR₅R₆, wherein R₅ and R₆ are C₁₋₁₀ alkyl or cycloalkyl groups as defined above.

Useful aminocarbonyl groups are carbonyl groups substituted by ―NH₂, ―NHR₅, and ―NR₅R₆, wherein R₅ and R₆ are C₁₋₁₀ alkyl groups.

Optional substituents on any of the heteroaryl rings in Formula **I** include any one of halo, haloalkyl, aryl. heterocyclo, cycloalkyl, heteroaryl, alkyl, alkenyl, alkynyl, arylalkyl. arylalkenyl, arylalkynyl, heteroarylalkyl. heteroarylalkenyl, heteroarylalkynyl, cycloalkylalkyl, heterocycloalkyl. hydroxyalkyl, aminoalkyl, carboxyalkyl, alkoxyalkyl, nitro, amino, ureido. cyano, acylamino, hydroxy, thiol, acyloxy, azido, alkoxy, carboxy. aminocarbonyl, and alkylthiol groups mentioned above. Preferred optional substituents include: halo, haloalkyl, hydroxyalkyl. aminoalkyl. nitro, alkyl. alkoxy and amino.

Certain of the compounds of Formula **I** may exist as optical isomers and the invention includes both the racemic mixtures of such optical isomers as well as the individual entantiomers that may be separated according to methods that are well know to those of ordinary skill in the art.

Examples of pharmaceutically acceptable addition salts include inorganic and organic acid addition salts such as hydrochloride, hydrobromide. phosphate. sulphate, citrate, lactate, tartrate, maleate, fumarate, mandelate, acetic acid. dichloroacetic acid and oxalate.

Examples of prodrugs include esters or amides of the compounds Formula **I** with optional substitution including hydroxyalkyl or aminoalkyl. and these may be prepared by reacting such compounds with anhydrides such as succinic anhydride.

The compounds of this invention may be prepared using methods known to those skilled in the art.

Compositions within the scope of this invention include all compositions wherein the compounds of the present invention are contained in an amount that is effective to achieve its intended purpose. While individual needs vary, determination of optimal ranges of effective amounts of each component is within the skill of the art. Typically, the compounds may be administered to mammals, e.g. humans, orally at a dose of 0.0025 to 50 mg/kg, or an equivalent amount of the pharmaceutically acceptable salt thereof. per day of the body weight of the mammal being treated for insomnia. For intramuscular injection, the dose is generally about one-half of the oral dose.

The unit oral dose may comprise from about 0.01 to about 50 mg, preferably about 0.1 to about 10 mg of the compound. The unit dose may be administered one or more times daily as one or more tablets each containing from about 0.1 to about 10, conveniently about 0.25 to 50 mg of the compound or its solvates.

In addition to administering the compound as a raw chemical, the compounds of the invention may be administered as part of a pharmaceutical preparation containing suitable pharmaceutically acceptable carriers comprising excipients and auxiliaries which facilitate processing of the compounds into preparations which can be used pharmaceutically. Preferably, the preparations, particularly those preparations which can be administered orally and which can be used for the preferred type of administration. such as tablets, dragees, and capsules. and also preparations which can be administered rectally, such as suppositories, as well as suitable solutions for administration by injection or orally, contain from about 0.01 to 99 percent, preferably from about 0.25 to 75 percent of active compound(s), together with the excipient.

Also included within the scope of the present invention are the non-toxic pharmaceutically acceptable salts of the compounds of the present invention. Acid addition salts are formed by mixing a solution of the particular heteroaryl compound of the present invention with a solution of a pharmaceutically acceptable non-toxic acid such as hydrochloric acid, fumaric acid, maleic acid, succinic acid, acetic acid, citric acid, tartaric acid, carbonic acid. phosphoric acid. oxalic acid, dichloroacetic acid. and the like. Basic salts are formed by mixing a solution of the heteroaryl compound of the present invention with a solution of a pharmaceutically acceptable non-toxic base such as sodium hydroxide, potassium hydroxide, choline hydroxide, sodium carbonate and the like.

The pharmaceutical compositions of the invention may be administered to any animal that may experience the beneficial effects of the compounds of the invention. Foremost among such animals are mammals, e.g., humans, although the invention is not intended to be so limited.

The pharmaceutical compositions of the present invention may be administered by any means that achieve their intended purpose. For example, administration may be by parenteral, subcutaneous, intravenous, intramuscular, intraperitoneal, transdermal, or buccal routes. Alternatively, or concurrently, administration may be by the oral route. The dosage administered will be dependent upon the age, health, and weight of the recipient, kind of concurrent treatment. if any. frequency of treatment, and the nature of the effect desired.

The pharmaceutical preparations of the present invention are manufactured in a manner which is itself known, for example, by means of conventional mixing, granulating, dragee-making, dissolving, or lyophilizing processes. Thus, pharmaceutical preparations for oral use can be obtained by combining the active compounds, which may advantageously be micronized, with solid excipients, optionally grinding the resulting mixture and processing the mixture of granules, after adding suitable auxiliaries, if desired or necessary, to obtain tablets or dragee cores.

Suitable excipients are, in particular, fillers such as saccharides, for example lactose or sucrose, mannitol or sorbitol. cellulose preparations and/or calcium phosphates, for example tricalcium phosphate or calcium hydrogen phosphate, as well as binders such as starch paste. using, for example. maize starch. wheat starch, rice starch, potato starch, gelatin, tragacanth, methyl cellulose, hydroxypropylmethylcellulose, sodium carboxymethylcellulose. and/or polyvinyl pyrrolidone. If desired, disintegrating agents may be added such as the above-mentioned starches and also carboxymethyl-starch, crosslinked polyvinyl pyrrolidone, agar, or alginic acid or a salt thereof, such as sodium alginate. Auxiliaries are, above all. flow-regulating agents and lubricants, for example, silica, talc. stearic acid or salts thereof, such as magnesium stearate or calcium stearate, and/or polyethylene glycol. Dragee cores are provided with suitable coatings which, if desired, are resistant to gastric juices. For this purpose, concentrated saccharide solutions may be used, which may optionally contain gum arabic, talc, polyvinyl pyrrolidone, polyethylene glycol and/or titanium dioxide. lacquer solutions and suitable organic solvents or solvent mixtures. In order to produce coatings resistant to gastric juices, solutions of suitable cellulose preparations such as acetylcellulose phthalate or hydroxypropymethyl-cellulose phthalate, are used. Dye stuffs or pigments may be added to the tablets or dragee coatings, for example, for identification or in order to characterize combinations of active compound doses.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils. or liquid paraffin. In addition, stabilizers may be added.

Possible pharmaceutical preparations, which can be used rectally, include. for example, suppositories. which consist of a combination of one or more of the active compounds with a suppository base. Suitable suppository bases are, for example, natural or synthetic triglycerides, or paraffin hydrocarbons. In addition. it is also possible to use gelatin rectal capsules which consist of a combination of the active compounds with a base. Possible base materials include. for example. liquid triglycerides, polyethylene glycols or paraffin hydrocarbons.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400 (the compounds are soluble in PEG-400). Aqueous injection suspensions may contain substances which increase the viscosity of the suspension, and include, for example, sodium carboxymethyl cellulose. sorbitol, and/or dextran. Optionally, the suspension may also contain stabilizers.

The following examples are illustrative, but not limiting, of the method and compositions of the present invention. Other suitable modifications and adaptations of the variety of conditions and parameters normally encountered in clinical therapy and which are obvious to those skilled in the art are within the spirit and scope of the invention.

3α-Hydroxy-3β-methoxymethyl-5α- and 5β-pregnan-20-ones were prepared from (*3R*)-spiro[oxirane-2', 5α- or 5β-pregnan]-20-one and sodium methoxide as described by Hogenkamp, et al., "Synthesis and in Vitro Activity of 3β-Substituted-3α-hydroxypregnan-20-ones: Allosteric Modulators of the GABA_{A} Receptor." *J. Med. Chem.* 40:61-72 (1997). 21-Substituted steroids were prepared from the corresponding 21-bromo steroids which were synthesized from the 20-ketosteroids using Br₂ in MeOH with catalytic HBr.

### Example 1

### 3α-Hydroxy-21-(1'-imidazolyl)-3β-methoxymethyl-5α-pregnan-20-one

### 21-Bromo-3α-hydroxy-3β-methoxymethyl-5α-pregnan-20-one.

To a solution of 3α-hydroxy-3β-methoxymethyl-5α-pregnan-20-one (30.0 g, 82.9 mmol) in 900 mL of methanol stirring at rt was added 3 drops of a 48% aqueous HBr solution. Bromine (13.9 g, 87.1 mmol) was then added dropwise as a solution in 200 mL of methanol over 2 h during which the reaction was shielded from light. After an additional 30 min, TLC (1% acetone/methylene chloride) indicated the absence of starting material and the formation of a less polar product. The reaction was concentrated to approximately 300 mL. CH₂Cl₂ (400 mL) was then added and the reaction was poured into a separatory funnel containing 200 mL of water. The phases were separated and the aqueous phase was extracted with CH₂Cl₂ (3 x 100 mL). The organic phases were combined, washed with 200 mL of a saturated aqueous NaHCO₃ solution, dried over Na₂SO₄, and concentrated under reduced pressure affording the bromide as a pale yellow foam. No further purification was carried out. **3α-Hydroxy-21-(1'-imidazolyl)-3β-methoxymethyl-5α-pregnan-20-one.**

To a suspension of the bromide prepared above (36.7 g, 82.9 mmol) in 800 mL of CH₃CN was added imidazole (28.2 g, 415 mmol) and the reaction was heated to reflux under Ar. The reaction was complete after I hour at reflux (TLC, 95:4.5:0.5 CH₂Cl₂:MeOH:Triethylamine (TEA)). The reaction was cooled to room temperature and was then concentrated *in vacuo.* The resulting oil was dissolved in 600 mL of CH₂Cl₂, washed with a dilute NaHCO₃ solution (4 x 200 mL), dried over Na₂SO₄ and concentrated *in vacuo.* Purification via flash chromatography on silica gel eluting with 95:4.5:0.5 CH₂Cl₂:MeOH:TEA afforded 18 g of the title compound as a white solid, mp 185-187 °C (evacuated capillary). Anal Calcd. for C₂₆H₄₀N₂O₃: C, 72.86; H, 9.41; N, 6.54. Found: C, 72.64; H, 9.35; N, 6.42. ¹H NMR (300 MHz, CDCl₃) δ 7.40 (s, 1H), 7.08 (s. 1H), 6.84 (s. 1H), 4.72 (d, 1H. J = 17.7 Hz). 4.64 (d, 1H, J = 18 Hz), 3.39 (s, 3H), 3.18 (s, 2H), 2.57 (t, 1H, J = 8.7 Hz), 0.76 (s, 3H), 0.66 (s, 3H).

### Example 2

### 3α-Hydroxy-21-(1'-imidazolyl)-3β-methoxymethyl-5α-pregnan-20-one, hydrochloride salt.

Hydrochloric gas (Aldrich) was bubbled through a solution of 3α-hydroxy-21-(1'-imidazolyl)-3β-methoxymethyl-5α-pregnan-20-one (1.00 g, 2.33 mmol) dissolved in 35 mL of CH₂Cl₂ for 7 m. A white precipitate formed. The solvent was removed *in vacuo,* affording 1.10 g of the hydrochloride salt as a white solid, mp 230-233 °C. ¹H NMR (300 MHz, CDCl₃) δ 9.66 (s, 1H), 7.31 (s, 1H), 7.05 (s, 1H), 5.45 (d, 1H, J = 18 Hz), 5.26 (d, 1H, J = 18 Hz), 3.39 (s, 3H), 3.19 (s, 2H), 2.72 (t, 1H. J = 8.7 Hz), 0.76 (s, 3H), 0.70 (s, 3H).

### Example 3

### 3α-Hydroxy-21-(1'-imidazolyl)-3β-methoxymethyl-5β-pregnan-20-one

To a solution of 3α-hydroxy-3β-methoxymethyl-5β-pregnan-20-one (2.0 g, 5.53 mmol) in 100 mL of MeOH was added one drop of a 48% aqueous HBr solution. followed by a solution of bromine (955 mg, 5.97 mmol) in MeOH added dropwise over 1 h. TLC (2% acetone/CH₂Cl₂) indicated complete reaction. The reaction was diluted with 50 mL of CH₂Cl₂ and partitioned between 100 mL each of CH₂Cl₂ and a sat. aq. NaHCO₃ solution. The aqueous layer was separated and washed with CH₂Cl₂ (3 x 25 mL). The pooled organic layers were dried (Na₂SO₄) and conc. in vacuo. The resulting residue was dissolved in CH₃CN (100 mL) and treated with solid imidazole (5 eq.; 1.88 g, 27.6 mmol). After 1 h at reflux, the reaction was allowed to cool and concentrated to dryness. The residue was partitioned between CH₂Cl₂ and a sat. aq. NaHCO₃ solution. The aqueous layer was separated and washed with CH₂Cl₂ (3 x 25 mL). The pooled organic layers were dried (Na₂SO₄) and conc. in vacuo. Purification via flash chromatography on silica gel eluting with 95:4.5:0.5 CH₂Cl₂:MeOH:TEA afforded 1.9 g of the title compound as a solid. ¹H NMR (CDCl₃, 300 MHz) δ 7.42 (s, 1H), 7.10 (s, 1H), 6.86 (s, 1H), 4.69 (m, 2H), 3.40 (m, 5H), 2.57 (t, 1H), 0.94 (s. 3H), 0.67 (s, 3H).

### Example 4

### 3α-Hydroxy-3β-methoxymethyl-21-(2'-tetrazolyl)-5α-pregnan-20-one

21-Bromo-3α-hydroxy-3β-methoxymethyl-5α-pregnan-20-one (1.70 g, 3.85 mmol), 1H-tetrazole (Aldrich; 0.27 g, 3.85 mmol) and potassium carbonate (2.60 g, 19.3 mmol) in anhydrous THF (15 mL) were heated at reflux overnight under Ar. The mixture was then partitioned between water (50 mL) and EtOAc (75 mL). The organic layer was separated, washed with water, dried over Na₂SO₄, and evaporated. The residue was purified by chromatography on silica gel, eluting with EtOAc/hexane (1:1), affording 830 mg (50 %) of the title compound, mp 165-167 °C. ¹H NMR (300 MHz, CDCl₃) δ 8.56 (s, 1H), 5.45 (s, 2H), 3.39 (s, 3H), 3.19 (s, 2H), 0.77 (s, 3H), 0.71 (s, 3H).

### Example 5

### 21-(5'-Amino-[1,3,4]-thiadiazol-2-ylthio)-3α-hydroxy-3β-methoxymethyl-5α-pregnan-20-one

21-Bromo-3α-hydroxy-3β-methoxymethyl-5α-pregnan-20-one (4.00 g, 9.72 mmol) was dissolved in 200 mL of acetonitrile and solid 5-amino-[1,3,4]-thiadiazol-2-thiol (1.42 g, 10.7 mmol) was added in one portion. The addition of neat triethylamine (1.49 mL, 10.7 mmol) gave a clear solution. After stirring at rt for 30 min, a white precipitate had formed and TLC (3:1 hexane:acetone) showed complete reaction. The mixture was cooled to 0 °C and the precipitate was isolated by filtration and washed with acetonitrile. The solid obtained was dried under vacuum affording 3.86 g (80%) of the title compound as a white solid, mp 169-172 °C. ¹H NMR (CDCl₃): δ 5.07 (bs, 2H), 4.11 (s, 2H), 3.39 (s, 3H), 3.18 (s, 2H), 2.74 (t, 1H), 0.75 (s, 3H), 0.64 (s, 3H). Anal. Calcd. for C₂₅H₃₉N₃O₃S₂: C, 60.82; H, 7.96; N, 8.51; S 12.99. Found: C, 60.70; H. 7.79; N, 8.51; S, 12.67.

### Example 6

### 3α-Hydroxy-3β-methroxymethyl-21-(quinolin-6-yloxy)-5α-pregnan-20-one, N-oxide

### 3α-Hydroxy-3β-methoxymethyl-21-(quinolin-6-yloxy)-5α-pregnan-20-one.

To a suspension of 6-hydroxyquinoline (Acros, 99+%; 4.74 g, 32.6 mmol) in 600 mL of acetonitrile at rt was added a 1.0 M solution of potassium *tert*-butoxide in THF (32.6 mL, 32.6 mmol). After stirring for 15 m, the 21-bromide prepared in example 2 (12.0 g, 27.2 mmol) was added as a solid and the reaction was allowed to stir at rt overnight. Analysis by TLC (1:1 hexane/ethyl acetate) indicated the complete consumption of the bromide and the formation of a much more polar, UV active product. Water (~750 mL) was added and the resulting mixture was stirred for 15 m. The suspension was vacuum filtered affording the title compound (12.6 g, 91%) as a tan solid, mp 178-180 °C. A sample of this material was submitted for combustion analysis with the following results: Calcd for C₃₂H₄₃NO₄-1/8 H₂O: C. 75.67; H. 8.58; N, 2.76. Found: C, 75.31; H, 8.74: N, 2.63.

### 3α-Hydroxy-3β-methoxymethyl-21-(quinolin-6-yloxy)-5α-pregnan-20-one N-oxide.

To a solution of the quinoline prepared above (12.0 g, 23.7 mmol) in 400 mL of dichloromethane was added 3-chloroperoxybenzoic acid (Aldrich, 57-83%; 6.53 g, ~26 mmol) and the resulting solution was stirred at rt overnight. TLC (1:1 dichloromethane/ethyl acetate) indicated complete consumption of the quinoline and formation of a much more polar product. The reaction was transferred to a separatory funnel and washed with a saturated aqueous NaHCO₃ solution (3 x 250 mL). The pooled organic layers were dried over Na₂SO₄ and concentrated *in vacuo.* The resulting orange solid was triturated with 100 mL each of hexane and acetonitrile overnight. Vacuum filtration of the mixture gave the product (9.59 g, 78%) as a light tan solid, mp softens at 180 °C, melts 197-200 °C. A sample of this material was submitted for combustion analysis with the following results: Calcd for C₃₂H₄₃NO₅-1/2 H₂O: C, 72.42; H, 8.35; N, 2.64. Found: C. 72.40; H, 8.48; N, 2.44. Recrystallization from EtOAc/MeOH gave the title compound as light tan prisms, mp 210-212 °C (evacuated capillary). ¹H NMR (300 MHz, CDCl₃) δ 8.68 (d, 1H, J = 9.6 Hz), 8.39 (d, 1H, J = 6.3 Hz), 7.59 (d, 1H, J = 8.4 Hz), 7.44 (dd, 1H, J = 2.6, 9.4 Hz), 7.24 (m, 1H), 7.00 (d, 1H, J = 2.4 Hz), 4.71 (d, 1H, J = 16.5 Hz), 4.62 (d, 1H, J = 16.5 Hz), 3.39 (s, 3H), 3.18 (s, 2H), 2.83 (t, 1H), 0.76 (s, 3H), 0.70 (s, 3H).

### Example 7

### Duration of action of 3α-hydroxy-3β-methoxymethyl-substituted steroids

Table I below compares the *in vitro* potencies [ability to inhibit the binding of [³⁵S]-*tert*-butylbicyclophosphorothionate (TBPS)], rotorod TD₅₀'s (dose at which half of animals tested fail to stay on a rotating rod for 1 minute) and the length of time before all animals tested are able to pass rotorod test (duration of action) of closely structurally related pairs of 3β-methyl and 3β-methoxymethyl steroids. These methods for measuring *in vitro* and *in vivo* activity of compounds of the invention are fully described in US patent 5,232,917. The TBPS assay gives the *in vitro* potency of compounds whereas the rotorod assay estimates the sedative/hypnotic activity of compounds. Since the duration of action of a compound is dependent on the dose and will be prolonged at higher doses, the duration of action was measured at the lowest dose where all of the animals failed the rotorod test. For compounds with duration of action > 240 minutes, the number of animals passing the rotorod test at 240 minutes is given in parentheses. In each pair, the 3β-methyl steroid has a biological duration action of greater than 240 minutes, while in each of the corresponding 3β-methoxymethyl steroids the duration of action is reduced to 180 minutes or less. In addition, the 3β-methyl steroids show less than half of the animals passing the rotorod at 240 minutes, suggesting a duration of action significantly longer. In two of the pairs of 3β-methoxymethyl and 3β-methyl steroids listed in Table 1, the former have a shorter duration of action than the latter despite being two-fold more potent *in vitro.* Thus, specific 3β-methoxymethyl-substituted neuroactive steroids gave unique and unexpected pharmacokinetic profiles, making them especially useful as sedative/hypnotic and anesthetic agents.

**Table 1.**

| **Comparison of *in vitro* potencies and the biological duration of action of 3β-methyl and 3β-methoxymethyl steroids in rat**^{**a**} | | | | |
|---|---|---|---|---|
| Compound | 3β-Group | TBPS IC₅₀ (nM) | RR TD₅₀ po (mg/kg) | Duration of action (minutes) |
| 3α-Hydroxy-21-(1'-imidazolyl)-3β-methoxymethyl-5α-pregnan-20-one | MeOCH₂ | 138 | 28 | 140 |
| 3α-Hydroxy-21-(1'-imidazolyl)-3β-methyl-5α-pregnan-20-one | Me | 97 | 31 | >240 (3/8 passing) |
| 3α-Hydroxy-3β-methoxymethyl-21-(quinolin-6-yloxy)-5α-pregnan-20-one, N-oxide | MeOCH₂ | 25 | 29 | 84 |
| 3α-Hydroxy-3β-methyl-21-(quinolin-6-yloxy)-5α-pregnan-20-one, N-oxide | Me | 46 | 15 | >240 (1/8 passing) |
| 3α-Hydroxy-3β-methoxymethyl-21-(2*'*-tetrazolyl)-5α-pregnan-20-one | MeOCH₂ | 24 | 35 | 120 |
| 3α-Hydroxy-3β-methyl-21-(2'-tetrazolyl)-5α-pregnan-20-one | Me | 44 | 4.5 | >240 (0/8 passing) |
| 21-(5-Amino-[1,3,4]-thiadiazol-2-ylthio)-3α-hydroxy-3β-methoxymethyl-5α-pregnan-20-one | MeOCH₂ | 48 | 40 | <90 |
| 3α-Hydroxy-21-(1'-imidazolyl)-3β-methoxymethyl-5β-pregnan-20-one | MeOCH₂ | 174 | 30 | <180 |

| | | | | |
|---|---|---|---|---|
| ^{a}IC₅₀ is the dose of steroid inhibiting 50% of specific binding of [³⁵S]-*tert-* butylbicyclophosphorothionate (TBPS). RR TD₅₀ is the dose at which half of animals fail the rotorod test in rat. Duration of action, measured at the lowest dose where all animals failed the rotorod test, is the time required for all animals tested to once again pass the rotorod test. | | | | |

Having now fully described this invention, it will be understood by those of ordinary skill in the art that the same can be performed within a wide and equivalent range of conditions, formulations and other parameters without affecting the scope of the invention or any embodiment thereof. All patents and publications cited herein are fully incorporated by reference herein in their entirety.

## Claims

1. A compound of Formula I: 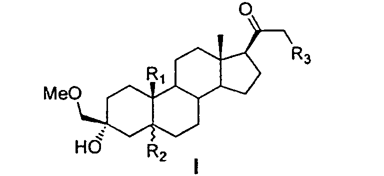 or a pharmaceutically acceptable salt, prodrug or solvate thereof, wherein:
wherein:
R₁ is H or methyl;
R₂ is 5α- or 5β-H;
R₃ is an optionally substituted *N*-attached heteroaryl group or a group -X-R₄;
R₄ is an optionally substituted carbon-attached heteroaryl group; and
X is O, S or N.

2. A compound of claim 1, wherein:
R₃ is an optionally substituted *N*-attached monocyclic heteroaryl group.

3. A compound of claim 1, wherein:
R₃ is -X-R₄;
R₄ is optionally substituted carbon-attached bicyclic heteroaryl group; and
X=O.

4. A compound of claim 2, wherein:
R₃ is optionally substituted (1'-imidazolyl) group or optionally substituted (2'-tetrazolyl) group.

5. A compound of claim 3, wherein:
R₄ is a carbon attached optionally substituted quinoline or isoquinoline or the corresponding *N*-oxide; and
X=O.

6. A compound of claim 1, wherein:
R₃ is -X-R₄;
R₄ is a carbon attached monocyclic heteroaryl group; and
X = S.

7. A compound of claim 4, which is 3α-hydroxy-21-(1'-imidazolyl)-3β-methoxymethyl-5α-pregnan-20-one or 3α-hydroxy-21-(1'-imidazolyl)-3β-methoxymethyl-5β-pregnan-20-one or a pharmaceutically acceptable salt thereof.

8. A compound of claim 4, which is 3α-hydroxy-3β-methoxymethyl-21-(2'-tetrazolyl)-5α-pregnan-20-one.

9. A compound of claim 5, which is 3α-hydroxy-3β-methoxymethyl-21-(quinolin-6-yloxy)-5α-pregnan-20-one, *N*-oxide.

10. A compound of claim 6, which is 21-(5'-amino-[1,3,4]-thiadiazol-2-ylthio)-3α-hydroxy-3β-methoxymethyl-5α-pregnan-20-one.

11. A pharmaceutical composition comprising the compound of claim I and a pharmaceutically acceptable carrier.

12. A method of alleviating or preventing insomnia in an animal subject, comprising administering to said animal subject in need of such treatment an effective amount of a compound in claim 1.

13. A method of inducing anesthesia in an animal subject in need of such treatment comprising administering to said animal subject in need of such treatment an effective amount of a compound in claim 1.
